Europäisches Patentamt

(19) European Patent Office

Office européen des brevets

(11) Veröffentlichungsnummer : **0 036 415**
**B1**

(12) **EUROPÄISCHE PATENTSCHRIFT**

(45) Veröffentlichungstag der Patentschrift :
04.07.84

(21) Anmeldenummer : **80901717.1**

(22) Anmeldetag : **17.09.80**

(86) Internationale Anmeldenummer :
**PCT/CH 80/00107**

(87) Internationale Veröffentlichungsnummer :
**WO/8100963 (16.04.81 Gazette 81/09)**

(51) Int. Cl.³ : **A 61 K 37/12, C 08 H 1/00,
C 09 H 1/00**

(54) **VERFAHREN ZUR HERSTELLUNG EINES KOLLAGENPRODUKTES FÜR MEDIZINISCHE UND KOSMETISCHE ZWECKE.**

(30) Priorität : **08.10.79 CH 9030/79**

(43) Veröffentlichungstag der Anmeldung :
**30.09.81 Patentblatt 81/39**

(45) Bekanntmachung des Hinweises auf die Patenterteilung : **04.07.84 Patentblatt 84/27**

(84) Benannte Vertragsstaaten :
**FR**

(56) Entgegenhaltungen :
DE-A- 1 692 548
DE-A- 2 625 289
DE-C-  673 203
FR-A- 2 108 068
FR-A- 2 369 329
GB-A- 1 144 552
US-A- 4 066 083
**Die Akte enthält technische Angaben, die nach dem
Eingang der Anmeldung eingereicht wurden und die
nicht in dieser Patentschrift enthalten sind.**

(73) Patentinhaber : **Pentapharm A.G.
Engelgasse 109
CH-4052 Basel (CH)**

(72) Erfinder : **RIES, Peter E.
Unterer Rebbergweg 123
CH-4153 Reinach BL (CH)**

(74) Vertreter : **Phélip, Bruno et al
c/o Cabinet Harlé & Phélip 21, rue de la Rochefoucauld
F-75009 Paris (FR)**

# 0 036 415

**Beschreibung**

## Technisches Gebiet

Die vorliegende Erfindung betrifft die Herstellung eines Kollagenproduktes mit filz-, schwamm- oder vliesartiger Struktur für medizinische und kosmetische Zwecke, welches im Vergleich zu bekannten Kollagenprodukten deutlich verbesserte physikalisch-chemische und mechanische Eigenschaften (Saugfähigkeit, Festigkeit) aufweist.

## Zugrundeliegender Stand der Technik

Es ist an sich bekannt, aus tierischen Geweben hergestellte Kollagenprodukte in der Medizin zur Wundversorgung 10 und zum Ausfüllen von pathologischen Knochenhohlräumen zu verwenden. Kollagenprodukte dieser Art sind z. B. in den folgenden Patenten beschrieben : Brit. Patent Nr. 1 147 072, Süd-afrikan. Patent Nr. 6 705 871, US Patent Nr. 4 016 877, US Patent Nr. 3 823 212, US Patent Nr. 3 810 473 und US Patent 15 Nr. 4 066 083 (P. Ries).

In der französischen Patentanmeldung Nr 2 108 068 ist ein Verfahren zur Herstellung eines Verbandmaterials aus Kollagen in Form eines ionisierbaren Partialsalzes beschrieben. Das Partialsalz wird durch Behandlung von nativen Kollagenfasern mit wässrigen Säuren, z. B. wässriger Salzsäure, erhalten.

In der britischen Patentschrift Nr. 1 144 552 ist ein Verfahren zur Herstellung eines faserigen Formkörpers durch Gefriertrocknen einer Dispersion eines ionisierbaren Salzes des Kollagens in Gegenwart einer wässrigen Säure, z. B. wässriger Salzsäure, beschrieben.

Bei den beiden oben erwähnten Verfahren wird als Ausgangsmaterial ein Salz oder Partialsalz des Kollagens verwendet.

Das im US Patent Nr. 4 066 083 beschriebene Verfahren besteht darin, dass man kollagenhaltiges tierisches Gewebe bei 40 °C nicht übersteigenden Temperaturen fein zerkleinert, den erhaltenen Gewebebrei durch wiederholte Behandlung mit dem 5-fachen Volumen, bezogen auf das Volumen des Gewebebreies, einer 5 bis 15 %igen wässrigen Natriumchloridlösung, die 0,2 bis 1 Gew.teil Natriumazid als Konservierungsmittel pro 1 000 Gew.teile der Lösung und 0,5 bis 2 Gew.teile eines nicht-ionischen fettdispergierenden Netzmittels enthält, entfettet und von unerwünschten wasserlöslichen nicht-kollagenartigen Ballaststoffen befreit, den erhaltenen Faserbrei mit Wasser oder 0,1 bis 0,5 %iger Ameisen-, Essig- oder Zitronensäure wäscht, den Faserbrei während 8 bis 48 h bei einem pH von 2,5 bis 3,5 in dem 5-fachen Volumen, bezogen auf das Volumen des Breies, 0,1-5 %iger wässriger Essigsäure, die 1 Gew.teil Pepsin auf 1 000 Gew.teile des als Ausgangsmaterial verwendeten Gewebes enthält, digeriert, um nicht-kollagenartige Proteine und Telopeptide zu entfernen, aus der erhaltenen Kollagensuspension durch Zugabe von Natriumchlorid in solcher Menge, dass die Natriumchloridkonzentration der Suspension 3 bis 5 % beträgt, Kollagen ausfällt, das gefällte Kollagen isoliert und durch Ultrafiltration, Dialyse oder Waschen mit 60 bis 75 %igem wässrigem Aethylalkohol weitgehend entsalzt, das entsalzte Kollagen in demineralisiertem oder destilliertem Wasser, welches bis zu 3 Gew.% einer starken organischen Säure enthält, in einem solchen Mengenverhältnis löst, dass die Kollagenkonzentration der erhaltenen Lösung einem Trockenrückstand von 0,5 bis 2 Gew.% entspricht, die Kollagenlösung gefriertrocknet und das gefriergetrocknete Kollagenprodukt sterilisiert.

Das nach diesem Verfahren erhaltene Kollagenprodukt besitzt eine Reihe günstiger Eigenschaften, welche es als Hilfsmittel in der Medizin besonders geeignet erscheinen lassen, weist jedoch den Nachteil auf, dass es wässrige Lösungen oder Körperflüssigkeiten langsam aufnimmt und im feuchten oder nassen Zustand eine ungenügende mechanische Festigkeit aufweist.

## Offenbarung der Erfindung

Es wurde nun überraschenderweise gefunden, dass man durch eine Wärmebehandlung des gefriergetrockneten Kollagenproduktes oder dessen Behandlung mit gasförmigem Halogenwasserstoff, vor oder nach der Sterilisation, die physikalisch-chemischen und mechanischen Eigenschaften des Kollagenproduktes wesentlich verbessern kann. Das verbesserte Kollagenprodukt ist für medizinische Zwecke noch geeigneter und leichter anwendbar. Wegen seiner verbesserten physikalisch-chemischen und mechanischen Eigenschaften ist das neue Kollagenprodukt auch als Kosmetikmaske für die Hautpflege verwendbar.

Gemäss der Erfindung kann das Kollagenprodukt nach dem Gefriertrocknen, vor oder nach der Sterilisation, einer Wärmebehandlung bei Temperaturen von 80 bis 150 °C, vorzugsweise 100 bis 130 °C, während 1/4 bis 12 h, vorzugsweise 1 bis 6 h, bei Normaldruck oder im Vakuum unterworfen werden. Man kann das gefriergetrocknete Kollagenprodukt, entweder vor oder nach der Sterilisation, auch einer Behandlung mit gasförmigem Halogenwasserstoff, vorzugsweise Chlorwasserstoff, während 1 bis 60 Min., vorzugsweise 5 Min., unterwerfen. Durch diese Behandlungen werden die Saugfähigkeit und die mechanische Nassfestigkeit des Kollagenproduktes erhöht. Gleichzeitig erzielt man eine Reduktion der Keimzahl im Kollagenprodukt. Das in der oben beschriebenen Weise behandelte Kollagenprodukt nimmt

2

innerhalb 1 Min. das 50- bis 100-fache seines Gewichtes an Wasser auf, während das gemäss dem US Patent Nr. 4 066 083 hergestellte, nicht zusätzlich behandelte Kollagenprodukt nur das 25- bis 30-fache seines Gewichtes an Wasser innerhalb 10 Min. aufnehmen kann. Das erfindungsgemäss hergestellte Kollagenprodukt weist auch nach der Aufnahme von Wasser oder Körperflüssigkeiten eine erhöhte mechanische Festigkeit auf, so dass es auch im nassen Zustand gut gehandhabt werden kann.

## Bester Weg zur Durchführung der Erfindung

Das zur Durchführung des erfindungsgemässen Verfahrens benötigte Ausgangsmaterial kann in der nachstehend beschriebenen Weise hergestellt werden :

1 kg Rindersehnen wurde bei − 10 bis − 20 °C eingefroren und während 10-20 Min. mit einem Messerhomogenisator hoher Tourenzahl sehr fein zerkleinert. Die Temperatur des Mahlgutes wurde durch Zugabe von Eisstückchen unter + 40 °C gehalten. Der erhaltene zähe fasrige Gewebebrei wurde in 5 Liter 10 %iger NaCl-Lösung, die 2,5 g Natriumazid und 50 ml einer 10 %igen wässrigen Lösung des nicht-ionischen Netzmittels NP 55/52 (Polyoxyäthylennonylphenyläther) enthielt, unter kräftigem Rühren suspendiert. Die Suspension wurde 2 h bei Raumtemperatur weitergerührt und anschliessend zentrifugiert. Der grau bis bräunlich gefärbte trübe Ueberstand, der Fett und unerwünschte wasserlösliche Ballaststoffe enthielt, wurde verworfen. Der zurückbleibende weisse Hautfaserbrei wurde noch zweimal in der gleichen Weise extrahiert, wobei der Extraktionsflüssigkeit pro Liter 0,1 Mol Dinatriumhydrogenphosphat zugesetzt wurde.

Als Ausgangsmaterial kann man anstelle von Rindersehnen auch Schweinehaut verwenden.

Ein in der oben beschriebenen Weise aus 1 kg Rindersehnen erhaltener entfetteter und extrahierter fasriger Gewebebrei wurde im 5-fachen Volumen 0,5 M Essigsäure suspendiert. Der Suspension wurde eine Lösung von 1 g technischem Pepsin in 100 ml 0,01 N HCl zugesetzt. Das pH der Suspension wurde mit HCl auf 2,9 eingestellt. Die Suspension wurde unter wiederholtem Rühren während 48 h digeriert. Die zähflüssige Kollagenlösung wurde durch einen Saugfilter G 1 filtriert, um undigierte Rückstände zu entfernen. Das Kollagen wurde durch Zugabe von 30 %iger wässriger Natriumhydroxydlösung aus der Suspension ausgefällt und durch Zentrifugieren abgetrennt. Das Kollagen wurde durch Lösen in 0,5 M Essigsäure und Ausfällen durch langsames Zugeben von 3 % Natriumchlorid gereinigt. Das gereinigte Kollagen wurde in 0,5 M Essigsäure gelöst und mit Wasser verdünnt. Das im Kollagen noch vorhandene restliche Natriumchlorid wurde durch Waschen auf einem Ultrafilter entfernt. Die Ultrafiltration wurde so lange fortgesetzt, bis im Eluat bei Zugabe von Silbernitrat keine Chloridionen mehr nachweisbar waren und die Kollagenkonzentration etwa 1 % betrug. Die Kollagenlösung wurde filtriert und schliesslich gefriergetrocknet.

## Beispiel 1

Eine nach der oben beschriebenen Methode hergestellte Kollagenlösung wurde in eine hitzebeständige Form gegossen und in einem Lyophilisator lyophilisiert, wobei gegen Ende der Lyophilisation die Temperatur auf 98 °C erhöht und der Trocknungsprozess 6 h bei dieser Temperatur fortgesetzt wurde. Nach Abkühlung wurde das Vakuum gebrochen und das trockene Kollagenprodukt verpackt und durch Bestrahlen mit γ-Strahlen sterilisiert.

Ein auf diese Weise hergestelltes Kollagenvlies von etwa 60 × 35 × 7 mm Grösse und 220 mg Gewicht nimmt innerhalb von 42 Sek. 15 ml Wasser auf. Zum Vergleich braucht ein gleich grosses und gleich schweres unbehandeltes Kollagenvlies 9 Min., um nur 5 ml Wasser aufzunehmen. Bei der Durchfeuchtung wird das nicht wärmebehandelte Kollagenvlies breiig weich, so dass es nicht mehr angefasst werden kann, ohne zu zerfallen, während das wärmebehandelte Kollagenvlies geschmeidig und im nassen Zustand für mindestens 1/2 h formbeständig und handhabbar bleibt.

## Beispiel 2

Ein nach der oben beschriebenen Methode hergestelltes gefriergetrocknetes Kollagenprodukt wurde vor Gebrauch in einem normalen, elektrisch beheizten Trockenschrank während 1 1/2 h auf 100 °C erhitzt und nach dem Erkalten auf Saugvermögen, Sauggeschwindigkeit und mechanische Nassfestigkeit, wie dies im Beispiel 1 beschrieben ist, geprüft. Das erhaltene Produkt zeigte die gleichen qualitativ verbesserten Eigenschaften wie das gemäss Beispiel 1 erhaltene Produkt.

## Beispiel 3

Gleiche Mengen einer nach der oben beschriebenen Methode hergestellten Kollagenlösung wurden in hitzebeständige, gleichartige Formen gegossen und zu Kollagenvliesen lyophilisiert. Die Hälfte der Vliese wurde in der im Beispiel 1 beschriebenen Weise einer Hitzebehandlung unterworfen. Die behandelten und die unbehandelten Vliese wurden in 5 cm breite Musterstreifen geschnitten und mit je 12 ml Wasser befeuchtet. Die Nassreissfestigkeiten der Muster wurden in einer Reissapparatur unter gleichen Bedingungen gemessen. Wie aus der nachfolgenden Tabelle ersichtlich ist, ist die Nassreissfestigkeit der behandelten Kollagenvliese signifikant höher als die der unbehandelten Vliese. Die Vliese, die zusätzlich 6 h bei 90 °C behandelt worden waren, rissen erst bei einer Belastung von 60 g bis 92 g, während die unbehandelten Vliese schon bei einer Belastung von 14 g bis 22 g rissen.

| Muster Nr. | Kollagennassreissfestigkeit | |
| | unbehandelt | behandelt |
|---|---|---|
| 1 | 22 g Belastung | 92 g Belastung |
| 2 | 14 g Belastung | 60 g Belastung |
| 3 | 14 g Belastung | 64 g Belastung |
| 4 | 14 g Belastung | 82 g Belastung |
| 5 | 18 g Belastung | 72 g Belastung |
| 6 | 18 g Belastung | 66 g Belastung |
| 7 | 18 g Belastung | 76 g Belastung |
| 8 | 20 g Belastung | 66 g Belastung |
| | $\varnothing$ = 17,25 g Belastung | $\varnothing$ = 72,25 g Belastung |

Zur Bestimmung der Nassreissfestigkeit wurde wie folgt verfahren :

Musterstreifen von Kollagenvlies von der Grösse 11 × 5 × 0,7 cm wurden zwischen zwei 6 cm breite Klammern im Abstand von 10 cm eingespannt. Der mittlere Teil der Muster wurde zuvor mit 12 ml destilliertem Wasser benetzt. Während die eine Klammer fixiert war, wurde die andere Klammer mit konstanter Geschwindigkeit weggezogen. Die bis zum Riss der Kollagenmuster aufgewendete Kraft wurde mit einem Dynamometer gemessen.

Beispiel 4

Nach der oben beschriebenen Methode hergestellte Kollagenvliesmuster von 5 × 11 × 0,7 cm Grösse wurden in zwei Gruppen A und B zu je 10 Vliesen unterteilt. Die Gruppe A wurde in einem Trockenofen 3 h auf 125 °C erhitzt, während Gruppe B unbehandelt blieb. Danach wurden die Nassreissfestigkeiten in der im Beispiel 3 beschriebenen Weise gemessen.

| Muster Nr. | Gruppe B | Gruppe A |
|---|---|---|
| 1 | 18 g Belastung | 120 g Belastung |
| 2 | 22 g Belastung | 142 g Belastung |
| 3 | 20 g Belastung | 162 g Belastung |
| 4 | 18 g Belastung | 132 g Belastung |
| 5 | 14 g Belastung | 162 g Belastung |
| 6 | 14 g Belastung | 146 g Belastung |
| 7 | 22 g Belastung | 164 g Belastung |
| 8 | 18 g Belastung | 148 g Belastung |
| 9 | 14 g Belastung | 150 g Belastung |
| Muster Nr. | Gruppe B | Gruppe A |
| 10 | 14 g Belastung | 140 g Belastung |
| | $\varnothing$ = 17,4 g Belastung | $\varnothing$ = 146,6 g Belastung |

Beispiel 5

Aus Kollagenvliesen, die in der im Beispiel 4 beschriebenen Weise hergestellt und erhitzt worden waren, wurden Muster von 200 bis 240 mg herausgeschnitten. Die Muster wurden auf ihre Saugfähigkeit gegenüber Wasser geprüft. Dazu wurde je ein Muster in eine flache Polystyrolschale gelegt und mit 16 ml Wasser versetzt. Nach 1 Min. wurden die Polystyrolschalen um 45° geneigt, wodurch das überschüssige, vom Kollagenvlies nicht aufgesogene Wasser ablief. Die gemessene Saugfähigkeit der hitzebehandelten Muster betrug das 50- bis 60-fache des Eigengewichtes, wie aus der folgenden Tabelle ersichtlich ist. Nach 24 h waren die nassen Kollagenvliesstücke immer noch formbeständig (stabil) und handhabbar.

4

| A Kollagengewicht | B Saugfähigkeit ohne Hitzebehandlung | C Saugfähigkeit nach Hitzebehandlung bei 125 °C während 3 h | Quotient 1 000 x C : A |
|---|---|---|---|
| 241 mg | 5,6 ml $H_2O$ | 13,9 ml $H_2O$ | 57,6 |
| 222 mg | 5,5 ml $H_2O$ | 11,7 ml $H_2O$ | 52,7 |
| 203 mg | 5,3 ml $H_2O$ | 12,3 ml $H_2O$ | 60,6 |
| 242 mg | 5,6 ml $H_2O$ | 13,9 ml $H_2O$ | 57,4 |

## Beispiel 6

Ein nach der oben beschriebenen Methode hergestelltes Kollagenvlies wurde in einen Exsiccator eingeschlossen, der an Stelle eines Trocknungsmittels ein Gefäss mit konzentrierter Salzsäure enthielt. Das Kollagenvlies wurde 5 Min. in der Chlorwasserstoffatmosphäre gelassen, dann herausgenommen, 1 h an der Luft stehen gelassen und dann auf Saugvermögen, Sauggeschwindigkeit und mechanische Nassreissfestigkeit, wie im Beispiel 1 beschrieben, geprüft. Das so behandelte Kollagenvlies zeigte die gleichen qualitativ verbesserten Eigenschaften wie das gemäss Beispiel 1 hergestellte Produkt.

Das erhaltene Produkt wurde unter den im Beispiel 3 definierten Bedingungen auf Nassreissfestigkeit geprüft. Es wurden folgende Werte ermittelt:

| Muster Nr. | Kollagennassreissfestigkeit | |
|---|---|---|
| | unbehandelt | HCl-behandelt |
| 1 | 20 g Belastung | 76 g Belastung |
| 2 | 18 g Belastung | 70 g Belastung |
| 3 | 14 g Belastung | 72 g Belastung |
| 4 | 18 g Belastung | 60 g Belastung |
| mittel | 17,5 g Belastung | 69,5 g Belastung |

Es war an sich bekannt, die physikalisch-chemischen Eigenschaften von Kollagen- oder Gelatineprodukten auf chemischem Wege, z. B. durch Vernetzen mit Aldehyden, insbesondere Formaldehyd oder Glutaraldehyd, zu verbessern. Diese Behandlungsmethoden haben jedoch den Nachteil, dass die erhaltenen Produkte bei der medizinischen Implantation vom Körper nur sehr langsam oder überhaupt nicht resorbiert werden und zudem im Körper Entzündungen, Abwehrreaktionen oder die Bildung von Fremdkörperriesenzellen verursachen. Es war nun völlig unerwartet und unerklärlich, dass man die physikalisch-chemischen Eigenschaften der Kollagenprodukte durch eine einfache Wärme- oder HCl-Behandlung in der oben beschriebenen Weise signifikant verbessern konnte und dass die so behandelten Produkte bei der medizinischen Verwendung im Körper die oben genannten Nachteile nicht aufweisen. Dies war umso überraschender, als Kollagenlösungen bekanntlich sehr hitzelabil sind und bei 35 °C nicht übersteigenden Temperaturen verarbeitet werden müssen.

**Ansprüche**

1. Verfahren zur Herstellung von Kollagenprodukten für medizinische und kosmetische Zwecke, darin bestehend, dass man kollagenhaltiges tierisches Gewebe bei 40 °C nicht übersteigenden Temperaturen fein zerkleinert, den erhaltenen Gewebebrei durch wiederholte Behandlung mit dem 5-fachen Volumen, bezogen auf das Volumen des Gewebebreies, einer 5 bis 15 %igen wässrigen Natriumchloridlösung, die 0,2 bis 1 Gew.teil Natriumazid als Konservierungsmittel pro 1 000 Gew.teile der Lösung und 0,5 bis 2 Gew.teile eines nichtionischen fettdispergierenden Netzmittels enthält, gleichzeitig entfettet und von unerwünschten wasserlöslichen nicht-kollagenartigen Ballaststoffen befreit, den erhaltenen Faserbrei mit Wasser oder 0,1 bis 0,5 %iger Ameisen-, Essig- oder Zitronensäure wäscht, den Faserbrei während 8 bis 48 h bei einem pH von 2,5 bis 3,5 in dem 5-fachen Volumen, bezogen auf das Volumen des Breies, 0,1 bis 5 %iger wässriger Essigsäure, die 1 Gew.teil Pepsin auf 1 000 Gew.teile des als Ausgangsmaterial verwendeten Gewebes enthält, digeriert, um nicht-kollagenartige Proteine und Telopeptide zu entfernen, aus der erhaltenen Kollagensuspension durch Zugabe von wässrigem Natriumchlorid in solcher Menge, dass die Natriumchloridkonzentration der Suspension 3 bis 5 % beträgt, Kollagen ausfällt, das gefällte Kollagen isoliert und durch Ultrafiltration, Dialyse oder Waschen mit 60 bis 75 %-igem wässrigem Aethylalkohol weitgehend entsalzt, das entsalzte Kollagen in demineralisiertem oder destilliertem Wasser, welches bis zu 3 Gew.% einer flüchtigen starken organischen Säure enthält, in einem solchen Mengenverhältnis löst, dass die Kollagenkonzentration der erhaltenen Lösung einem Trockenrückstand von 0,5 bis 2 Gew.% entspricht, die Kollagenlösung gefriertrocknet und das gefriergetrocknete Kollagenprodukt sterilisiert, dadurch gekennzeichnet, dass man das Kollagenprodukt nach dem Gefriertrocknen, vor oder nach der Sterilisation, einer Wärmebehandlung oder einer Behandlung mit gasförmigem Halogenwasserstoff unterwirft.

2. Verfahren nach Anspruch 1, dadurch gekennzeichnet, dass man das gefriergetrocknete Kollagenprodukt 1/4 bis 12 h Temperaturen von 80 bis 150 °C aussetzt.

3. Verfahren nach Anspruch 1, dadurch gekennzeichnet, dass man das gefriergetrocknete Kollagenprodukt während 1 bis 60 Min. einer Halogenwasserstoffatmosphäre, z. B. einer Chlorwasserstoffatmosphäre, aussetzt.

4. Verfahren nach Anspruch 1, dadurch gekennzeichnet, dass man als tierisches Gewebe Schweinehaut verwendet.

5. Verfahren nach Anspruch 1, dadurch gekennzeichnet, dass man als tierisches Gewebe Rindersehnen verwendet.

## Claims

1. Process for the preparation of collagen products for medical and cosmetic purposes, which comprises reducting to fine particles, at temperatures which do not exceed 40 °C, animal tissue containing collagen, removing undesired water-soluble non-collagenous bulk materials and, at the same time, removing lipids from the resulting pulped tissue by a repeated treatment with five times the volume of the pulped tissue of a 5 to 15 % aqueous sodium chloride solution which contains 0.2 to 1 part by weight of sodium azide as a preservative per 1,000 parts by weight of the solution and contains 0.5 to 2 parts by weight of a non-ionic lipid-dispersing wetting agent, washing the resulting pulped fibres with water or with 0.1 to 0.5 % formic, acetic or citric acid, digesting the pulped fibres, for 8 to 48 h at a pH of 2.5 to 3.5, in five times the volume of the pulp of 0.1 to 5 % aqueous acetic acid which contains one part by weight of pepsin per 1,000 parts by weight of the tissue used as a starting material, in order to remove non-collagenous proteins and telopeptides, precipitating collagen from the resulting collagen suspension by adding aqueous sodium chloride in an amount such that the sodium chloride concentration in the suspension is 3 to 5 %, isolating and extensively desalting the precipitated collagen by ultrafiltration, dialysis or washing with a 60 to 75 % aqueous ethyl alcohol, dissolving the desalted collagen in demineralised or distilled water which contains up to 3 % by weight of a volatile strong organic acid, in a ratio such that the concentration of collagen in the resulting solution corresponds to a dry residue of 0.5 to 2 % by weight, freeze-drying the collagen solution and sterilising the freeze-dried collagen product, which process is characterised in that the collagen product is subjected, after freeze-drying but before or after sterilisation, to heat treatment or treatment with gaseous hydrogen halide.

2. Process according to claim 1, characterised in that the freeze-dried collagen product is exposed to temperatures of 80 to 150 °C for 1/4 to 12 h.

3. Process according to claim 1, characterised in that the freeze-dried collagen product is exposed for 1 to 60 min to an atmosphere of hydrogen halide, for example an atmosphere of hydrogen chloride.

4. Process according to claim 1, characterised in that the skin of pigs is used as the animal tissue.

5. Process according to claim 1, characterised in that the tendons of cattle are used as the animal tissue.

## Revendications

1. Procédé de préparation de produits à base de collagène pour usage médical et cosmétique, consistant à réduire en fines particules du tissu animal contenant du collagène, à des températures ne dépassant pas 40 °C, à éliminer les substances ballast hydrosolubles exemptes de collagène indésirables et, en même temps, les lipides de la bouillie de tissu obtenue par un traitement répété à l'aide de 5 fois le volume de la bouillie de tissu d'une solution aqueuse de chlorure de sodium à la concentration de 5 à 15 % qui contient comme agent de conservation, 0,2 à 1 partie en poids d'azoture de sodium pour 1 000 parties en poids de la solution et qui contient 0,5 à 2 parties en poids d'un agent mouillant non ionique dispersant les lipides, à laver la bouillie de fibres obtenue avec de l'eau ou de l'acide formique, acétique ou citrique à la concentration de 0,1 à 0,5 %, à digérer la bouillie de fibres pendant 8 à 48 h à un pH de 2,5 à 3,5 dans 5 fois le volume de la bouillie d'acide acétique aqueux à la concentration de 0,1 à 5 % contenant 1 partie en poids de pepsine pour 1 000 parties en poids du tissu utilisé comme matière première, afin d'éliminer les protéines et les télopeptides exemptes de collagène, à précipiter le collagène dans la suspension de collagène obtenue par addition de chlorure de sodium aqueux en quantité telle que la concentration en chlorure de sodium dans la suspension soit de 3 à 5 %, à isoler le collagène précipité et à les soumettre à une déminéralisation poussée par ultrafiltration, dialyse ou lavage avec de l'alcool éthylique aqueux à la concentration de 60 à 75 %, à dissoudre le collagène déminéralisé dans de l'eau déminéralisée ou distillée contenant jusqu'à 3 % en poids d'un acide fort volatil, dans un rapport quantitatif tel que la concentration en collagène de la solution obtenue corresponde à un résidu sec de 0,5 à 2% en poids, à lyophiliser la solution de collagène et à stériliser le produit au collagène lyophilisé, procédé caractérisé en ce que l'on soumet le produit au collagène, après lyophilisation mais avant ou après stérilisation, à un traitement thermique ou à un traitement avec un halogénure d'hydrogène gazeux.

2. Procédé selon la revendication 1, caractérisé en ce que le produit au collagène lyophilisé est exposé à des températures de 80 à 150°C pendant 1/4 d'heure à 12 heures.

3. Procédé selon la revendication 1, caractérisé en ce que le produit au collagène lyophilisé est exposé à une atmosphère d'halogénure d'hydrogène, par exemple une atmosphère de chlorure d'hydrogène, pendant 1 à 60 minutes.

4. Procédé selon la revendication 1, caractérisé en ce que l'on utilise la peau de porc comme tissu animal.

5. Procédé selon la revendication 1, caractérisé en ce que l'on utilise les tendons de bovins comme tissu animal.